(19) Europäisches Patentamt / European Patent Office / Office européen des brevets

(11) **EP 4 053 263 A1**

(12) **EUROPEAN PATENT APPLICATION**
published in accordance with Art. 153(4) EPC

(43) Date of publication:
**07.09.2022 Bulletin 2022/36**

(21) Application number: **21842680.7**

(22) Date of filing: **14.07.2021**

(51) International Patent Classification (IPC):
*C12N 5/071* (2010.01)   *A61L 27/38* (2006.01)
*A61K 35/42* (2015.01)   *A61P 11/00* (2006.01)

(52) Cooperative Patent Classification (CPC):
**A61K 35/42; A61L 27/38; A61P 11/00;
A61P 43/00; C12N 5/06**

(86) International application number:
**PCT/CN2021/106176**

(87) International publication number:
**WO 2022/012567 (20.01.2022 Gazette 2022/03)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **15.07.2020 CN 202010682604**

(71) Applicant: **Regend Therapeutics Co., Ltd.
Jiangsu 215000 (CN)**

(72) Inventor: **ZHANG, Ting
Suzhou, Jiangsu 215000 (CN)**

(74) Representative: **J A Kemp LLP
80 Turnmill Street
London EC1M 5QU (GB)**

(54) **NEW USE OF REGEND001 CELL AUTOLOGOUS REINFUSION PREPARATION IN TREATMENT OF IDIOPATHIC PULMONARY FIBROSIS**

(57)   The present invention provides a REGEND001 cell autologous deliverable formulation for use in improving the pulmonary function of a patient suffering from idiopathic pulmonary fibrosis. The preparation is prepared by collection, in vitro isolation and culture and amplification of the patient's autologous bronchial basal cells.

Figure 1

Description

TECHNICAL FIELD

The present invention relates to the field of clinical medicine, and particularly relates to a new use of a REGEND001 cell autologous deliverable formulation.

BACKGROUND

Idiopathic pulmonary fibrosis (IPF) is a chronic progressive fibrotic interstitial pneumonia with obscure etiology. IPF has been included in the 2018 National Rare Diseases List, and at present, its incidence is increasing annually in China. IPF tends to occur in middle- and old-aged male population and is manifested as progressively worsening dyspnea, accompanied by restrictive ventilatory dysfunction and gas exchange disorder, eventually leading to hypoxemia and even respiratory failure. The prognosis of IPF is poor, and its lung histology and high-resolution CT (HRCT) of the chest are manifested as a usual interstitial pneumonia (UIP) with diffuse alveolitis and pulmonary interstitial fibrosis as the main pathologic features, and progressive and irreversible lung damage. The lost of alveoli in numbers leads to the decline or even loss of the pulmonary ventilation function, resulting in hypoxia, reduced mobility and even death of patients. As a result, the average survival duration for those diagnosed with IPF is only 2.8 years, the mortality rate is higher than that of most tumors, and thus IPF is called a "tumor-like disease".

The traditional method for treatment of IPF is mainly administrating anti-inflammatory drugs and anti-fibrotic drugs. Glucocorticoids can suppress the inflammatory response and the immune process. Immunosuppressants (cyclophosphamide, azathioprine, methotrexate, etc.) also have the effect of inhibiting the inflammatory response. Thus, glucocorticoids and immunosuppressants/cytotoxic drugs were used as the basic drugs for the treatment of IPF, but they are not ideally effective in patients with intermediate to advanced fibrosis. The drugs currently approved for IPF treatment in China include Pirfenidone, Nintedanib, etc., which have a delay effect on the decline of pulmonary function of patients. But it is difficult to improve the pulmonary function or reverse the progression of the disease, and the occurrence of fibrosis cannot be really prevented. At present, the fundamental reason for the lack of effective conventional treatment for IPF worldwide, other than whole-lung transplantation, is the lack of effective methods to regenerate and repair the damaged alveolar structures.

The Frank McKeon research group at Harvard University was the first to identify a cell group of bronchial basal cells from the distal bronchi of mice in 2011. In 2018, such group of bronchial basal cells was successfully identified in adults *in vivo* and isolated and amplified *in vitro.* This group of cells belongs to the basal cell category and has the potential to differentiate into bronchial and alveolar epithelium.

SUMMARY

The present application provides a use of a REGEND001 autologous cell deliverable formulation in preparing a product, wherein the product has a function of improving the pulmonary function in patients with idiopathic pulmonary fibrosis. The product is, for example, a drug or pharmaceutical composition for the treatment of idiopathic pulmonary fibrosis.

The REGEND001 autologous cell deliverable formulation is a finished cell formulation that is prepared by collecting, isolating *in vitro,* culturing and amplification of autologous bronchial basal cells of patients. A preparation process of the REGEND001 autologous cell deliverable formulation may adopt the methods given in the Examples of the present application or the methods of preparing clinical-grade autologous bronchial basal cells and a deliverable formulation described in Chinese patent application 201910407062.7 (CN111944737A), which is hereby incorporated herein by reference.

The present application provides a use of the REGEND001 autologous cell deliverable formulation in the preparation of a product, wherein the product has a function of improving the pulmonary ventilation function in patients with idiopathic pulmonary fibrosis, and the product is, for example, a drug or pharmaceutical composition for improving the pulmonary ventilation function in patients with idiopathic pulmonary fibrosis.

The present application provides a use of the REGEND001 autologous cell deliverable formulation in the preparation of a product for improving and/or treating the pulmonary ventilatory function in humans.

The present application provides a use of the REGEND001 autologous cell deliverable formulation in the preparation of a product for improving the pulmonary diffusion function in humans.

The present application provides a use of the REGEND001 autologous cell deliverable formulation in the preparation of a product for improving the motor function in humans.

The above REGEND001 autologous cell deliverable formulation is administered to any pulmonary subsegment of the lingual lobe of the left lung, the inferior lobe of the left lung, the middle lobe of the right lung, and the inferior lobe of the right lung.

The REGEND001 autologous cell deliverable formulation is administered at a dose of 0.1-10×10$^6$ bronchial basal cells/kg, preferably 1-10×10$^6$ cells/kg.

The present application provides a use of the REGEND001 autologous cell deliverable formulation in the treatment of idiopathic pulmonary fibrosis.

In some embodiments, the REGEND001 autologous cell deliverable formulation contains bronchial basal cells suspended in normal saline for injection, and each 14 mL of the REGEND001 autologous cell deliverable formulation contains (4-300) × 10$^6$ bronchial basal cells, preferably (2-30) × 10$^7$ bronchial basal cells.

In some embodiments, the REGEND001 autologous cell deliverable formulation is prepared by the following steps:

tissue preparation: getting an ex vivo bronchoscopic brushed-off biopsy tissue from at least one site;

enzyme digestion: digesting the tissue with a tissue digestion solution, and collecting digested cells after terminating the digestion with a stop buffer;

plating and amplification culture: plating a part of the digested cells in a culture flask pre-coated with feeder cells to perform primary culture, collecting the primary cultured cells to perform amplification culture in a culture flask pre-coated with feeder cells, followed by passage culture when the cells grow to cover 50%-90% of the surface area of the culture flask, or when the cells grow to form clones and further form colonies wherein 80% or more of the clones contain 40-100 cells and clones of grade A and grade B are observed in three fields of view among randomly selected five fields of view; wherein the clones of grade A have a regular, round and smooth outline, and clear boundary, and cells are closely arranged in the clones and have uniform sizes; the clones of grade B have a roughly regular outline, and smooth and clear boundary, and most cells are closely arranged in clones and have uniform sizes, yet a small amount of the cells have a slightly larger size and are slightly loose arranged;

cell collecting: when passaged cells grow to cover 85%-95% the surface area of the culture flask, digesting and collecting adherent cells, and washing.

In some embodiments, the tissue digestion solution comprises 99 v% of DMEM/F12, 1-20 ng/mL of DNase, 0.1-4 mg/mL of proteinase type XIV, and 10-200 ng/mL of trypsin; the digesting is performed at a temperature of 37°C for a duration of 0.5-2 h.

In some embodiments, said primary culture and amplification culture are carried out with a culture medium comprising: 225 mL of DMEM, 225 mL of F12, 20-70 mL of fetal bovine serum (FBS), 0.2-2 mM of L-glutamine, 1-14 ng/mL of insulin, 0.1-1 ng/mL of epidermal growth factor, 5-30 μg/mL of adenine, and 2-20 μg/mL of hydrocortisone.

In some embodiments, the stop buffer comprises 90 v% of DMEM and 10 v% of FBS.

In some embodiments, the digested cells and the primary cells are cryopreserved; preferably before the cryopreservation, bacterial detection and mycoplasma detection are carried out; and preferably the cryopreserved cells are rapidly thawed and used to perform the primary culture and/or amplification culture.

According to one aspect of the present application, provided is a method for treating idiopathic pulmonary fibrosis, comprising administering REGEND001 autologous cell deliverable formulation to a subject with idiopathic pulmonary fibrosis, wherein the REGEND001 autologous cell deliverable formulation is a finished cell formulation prepared by collecting, isolating *in vitro,* and culturing and amplification of autologous bronchial basal cells of patients.

In some embodiments, the method for treating idiopathic pulmonary fibrosis includes delivering the REGEND001 autologous cell deliverable formulation to one or more of 14 pulmonary subsegments from the lingual lobe of the left lung, the inferior lobe of the left lung, the middle lobe of the right lung, and/or the inferior lobe of the right lung. The 14 pulmonary subsegments are the lateral segmental bronchus of the middle lobe of the right lung, the medial segmental bronchus of the middle lobe of the right lung, the apical segmental bronchus of the inferior lobe of the right lung, the medial basal segmental bronchus of the right lung, the anterior basal segmental bronchus of the right lung, the lateral basal segmental bronchus of the right lung, the posterior basal segmental bronchus of the right lung, the superior segmental bronchus of the lingual lobe of the left lung, the inferior segmental bronchus of the lingual lobe of the left lung, the apical segmental bronchus of the inferior lobe of the left lung, the anterior basal segmental bronchus of the left lung, the medial basal segmental bronchus of the left lung, the lateral basal segmental bronchus of the left lung, and the posterior basal segmental bronchus of the left lung, respectively.

In some embodiments, the REGEND001 autologous cell deliverable formulation comprises bronchial basal cells suspended in normal saline for injection, and each 14 mL of the REGEND001 autologous cell deliverable formulation contains (4-300) × 10$^6$ bronchial basal cells, preferably (2-30) × 10$^7$ bronchial basal cells.

In some embodiments, the method for treating idiopathic pulmonary fibrosis comprises diluting 14 mL of the REGEND001 autologous cell deliverable formulation into 42 mL with normal saline, and then injecting the diluted solution into one or more of the 14 pulmonary subsegments by using a syringe.

In some embodiments, the subject with idiopathic pulmonary fibrosis has moderate or severe diffusion dysfunction.

In some embodiments, the subject with idiopathic pulmonary fibrosis further suffers from one or more diseases or conditions selected from hypertension, type 2 diabetes, coronary artery disease, Hashimoto's thyroiditis, and pulmonary emphysema.

In some embodiments, the REGEND001 autologous cell deliverable formulation is administered at a dose of $0.1\text{-}10\times10^6$ bronchial basal cells/kg, preferably $1\text{-}10\times10^6$ bronchial basal cells/kg.

The technical solutions of the present application have the advantages as follows: a bronchial basal cell product, namely the REGEND001 autologous cell deliverable formulation, is developed in the present application. In an early exploratory clinical study, the REGEND001 autologous cell deliverable formulation is autologously delivered into patients with idiopathic pulmonary fibrosis *via* local administration through airway, which shows the effects of repairing lung damage and improving the pulmonary ventilation function.

## BRIEF DESCRIPTION OF THE DRAWINGS

In order to more clearly illustrate the specific embodiments of the present application or the technical solutions in the prior art, the accompanying drawings required for use in the description of the specific embodiments or the prior art will be briefly described below. It will be apparent that the accompanying drawings in the following description are some embodiments of the present application, and that other drawings may be obtained from these drawings without any creative effort for a person of ordinary skill in the art.

Figure 1 shows the change in diffusion function (diffusing capacity of carbon monoxide (DLCO) actual measured value/predicted value) before and after treatment of Case 1 in Example 3 of the present application.

Figure 2 shows the change in diffusion function (DLCO actual measured value) before and after treatment of Case 1 in Example 3 of the present application.

Figure 3 shows the change in diffusion function (DLCO actual measured value/predicted value) before and after treatment of Case 2 in Example 3 of the present application.

Figure 4 shows the change in diffusion function (DLCO actual measured value) before and after treatment of Case 2 in Example 3 of the present application.

Figure 5 shows the change in diffusion function (DLCO actual measured value/predicted value) before and after treatment of Case 3 in Example 3 of the present application.

Figure 6 shows the change in diffusion function (DLCO actual measured value) before and after treatment of Case 3 in Example 3 of the present application.

## DETAILED DESCRIPTION

The DLCO predicted values in the following examples are calculated from an equation of predicted values recommended by the European Thoracic Society (ECSC). For Chinese, the predicted values are obtained by adding a correction factor for correction on the basis of the ECSC equation. The predicted

values are usually correlated with the height (H), weight (W), and age (A) of the patients. A formula for calculating a predicted value of the pulmonary function in adults in East China is:

$$DLCO = 0.3894 \times H - 0.1244 \times A - 0.0790 \times W - 25.3405.$$

The reference standard for the 6-minute walk test in the following examples is described as follows:
according to the detailed guidelines for the 6-minute walk test published by the American Thoracic Society (ATS) in 2002, the 6-minute walk test is an exercise test of the functional status in patients with moderate or severe cardiopulmonary diseases and is primarily applicable to measure the response of patients with moderate to severe cardiac or pulmonary diseases to medical interventions, primarily to assess the patient's exercise capacity. There is no final conclusion on which way (an absolute value, a percentage, or a change/predicted value %) to express the change in the 6-minute walk distance has the greatest clinical significance, and the guideline recommend the use of the absolute value.

St. George's Respiratory Questionnaire (SGRQ) reference standard is described as follows:
the St. George Respiratory Questionnaire (SGRQ) is one of the most widely used specific scales for measuring health impairment and quality of life in adult patients with respiratory diseases, and is a standardized patient self-administered questionnaire scored by weighting based on questions in symptoms (frequency and severity of symptoms), activity (capability of causing shortness of breath or restriction of movement caused by shortness of breath), and impact on daily activities (impairment of social competence and psychological disorder due to airway disease). The higher the score, the worse the quality of life (reference: Jones PW, Quirk FH, Baveystock CM. The St George's Respiratory Questionnaire. Respir Med. 1991 Sep; 85 Suppl B:25-31; discussion 33-7). The SGRQ is currently adopted by more and more respiratory diseases to assess the quality of life of patients. This evaluation index was also used in the clinical trial of Nintedanib, a chemical drug approved in 2017 for the treatment of IPF. There are currently no fixed criteria, it is mainly being used to compare patients themselves before and after treatment.

Example 1 Preparation of REGEND001 autologous cell deliverable formulation

This example provides a process for preparing clinical-grade autologous bronchial basal cells, specifically including the following steps:

ex vivo bronchoscopic brushed-off biopsy tissues were prepared, and in this example, tissues from two different sites of the bronchus were selected. Selecting tissues from two or more sites is to ensure the success rate of separation.

A tissue digestion solution and a stop buffer were prepared. The tissue digestion solution comprised 99 v% of DMEM/F12, 1-20 ng/mL of DNase, 0.1-4 mg/mL of protease type XIV, and 10-200 ng/mL of trypsin. The stop buffer comprised 90 v% of DMEM and 10 v% of FBS.

The brushed-off biopsy tissues were digested with the tissue digestion solution. The digestion was terminated with the stop buffer, and digested cells were collected. The cells after enzyme digestion were subjected to bacterial detection and mycoplasma detection, and then part of the digested cells were cryopreserved.

A culture medium was prepared, wherein the culture medium comprised 225 mL of DMEM, 225 mL of F12, 20-70 mL of FBS, 0.2-2 mM of L-glutamine, 1-14 ng/mL of insulin, 0.1-1 ng/mL of epidermal growth factor, 5-30 $\mu$g/mL of adenine, and 2-20 $\mu$g/mL of hydrocortisone.

Irradiation-inactivated feeder cells were plated in T25 cell culture flasks and cultured in a cell incubator (37°C, 5% $CO_2$) for 1-2 days, wherein the cells should have a density that the cells covered 50-70% of the bottom surface of the culture flask after 1-2 days.

Some of the digested cells were taken, and the cells derived from respective site were resuspended and plated into one T25 culture flask pre-coated with feeder cells, 200ng/mL of gentamicin was added, and the cells were cultured at 37°C with a $CO_2$ concentration of 4%-8%. The culture medium was replaced every other day. The cells were collected and passaged when the cells grew to form clones, wherein 80% or more of the clones contained 40-100 cells and clones of grade A and grade B were observed in three fields of view among randomly selected five fields of view.

The clones can be classified as grades A, B, and C depending on the morphology. The clones of grade A have a regular, round and smooth outline, and clear boundary, and cells are closely arranged in clones and have uniform sizes. The clones of grade B have a roughly regular outline, smooth and clear boundary, and most cells are closely arranged in clones and have uniform sizes, yet a small amount of the cells have a slightly larger size and are slightly loose arranged. The clones of grade C have an irregular outline, and unclear boundary, and the cells are loosely arranged in clones and have large sizes.

First passage was carried out as follows: the cell culture supernatant was removed, and the cells were washed once with 1× DPBS, and digested with 2mL of recombinant trypsin at 37°C for 5-10 min. After most of the cells became round and bright, adherent cells were detached by pipetting up and down and prepared into a single-cell suspension. The digestion was terminated with an equal volume of DMEM medium, and the cell suspension was collected, and centrifuged at 1200 rpm for 5 min. Supernatant was removed, and the cells were resuspended in the culture medium. Finally the re-suspended cells were plated onto one T75 cell culture flask pre-coated with feeder cells at a density of 5-10×$10^3$ cells/cm$^2$. The culture medium was replaced every other day.

2nd passage was carried out when the first passaged cells grew to cover 50%-90% of the bottom surface area of the culture flask. The culture supernatant was removed, and the cells were washed once with 1×DPBS, and digested with 5mL of recombinant trypsin at 37°C for 5-10 min. Adherent cells were detached by pipetting up and down and prepared into a single-cell suspension after most of the cells became round and bright. The digestion was terminated with an equal volume of DMEM medium, and the resulting cell suspension was collected and centrifuged at 1200 rpm for 5 min. Supernatant was removed, and the cells are re-suspended in the culture medium. The re-suspended cells were plated onto three T75 cell culture flasks pre-coated with feeder cells at a density of 5-10×$10^3$ cells/cm$^2$, and the culture medium was replaced every other day.

3rd passage was carried out when the second passaged cells grew to cover 50%-90% of the bottom surface area of the culture flasks. For cells in each flask, the culture supernatant was removed, and the cells were washed once with 1×DPBS, and digested with 5mL of recombinant trypsin at 37°C for 5-10 min. After most of the cells became round and bright, adherent cells were detached by pipetting up and down and prepared into a single-cell suspension. The digestion was terminated with an equal volume of DMEM medium. Cell suspension was collected and centrifuged at 1200 rpm for 5 min. Supernatant was removed, and the cells were resuspended in the culture medium. 2mL of cell culture supernatant and 3-16×$10^5$ cells were taken to carry out tests, wherein bacterial detection and mycoplasma detection gave negative results, gentamicin residual result was less than 5.4 ppb, HOPX positive percentage in biological potency test was ≥ 30%, cell identification test confirmed cells were KRT5 positive, and cell purity analysis showed KRT5 cell percentage was ≥ 90%. Meanwhile, the re-suspended cells were plated onto ten T75 cell culture flasks pre-coated with feeder cells at a density of $5-10\times10^3$ cells/cm$^2$, and the culture medium was replaced every other day.

The fourth passage was performed when the third passaged cells grew to cover 50%-90% of the bottom surface area of the culture flask. For cells in each flask, the culture supernatant was removed, and the cells were washed once with $1\times$DPBS, and digested with 5mL of recombinant trypsin at 37°C for 5-10 min. After most of the cells became round and bright, adherent cells were detached by pipetting up and down and prepared into a single-cell suspension. The digestion was terminated with an equal volume of DMEM medium. The cell suspension was collected, and centrifuged at 1200 rpm for 5 min. Supernatant was removed, and the cells were resuspended in the culture medium. $1-10\times10^5$ cells were taken for biological potency test, wherein the HOPX positive percentage was ≥30%. Meanwhile, the resuspended cells were plated on 30 T75 cell culture flasks pre-coated with feeder cells at a density of $5-10\times10^3$ cells/cm$^2$, and the culture medium was replaced every other day.

The feeder cells were removed when the fourth passaged cells grew to cover 50%-90% of the bottom surface area of the culture flask. The operation was performed as follows: the culture supernatant in each culture flask was removed, and each culture flask was washed once with $1\times$DPBS, 5mL of recombinant trypsin was added, and the culture flask was placed at 37 °C for 1-2 min. The feeder cells were detached by pipetting up and down, and each flask was washed again with $1\times$DPBS, and 5 mL of recombinant trypsin was added for digestion. After most of the cells became round and bright, adherent cells were detached by pipetting up and down and prepared into a single-cell suspension. The digestion was terminated with an equal volume of DMEM medium. The cell suspension was collected, and centrifuged at 1200 rpm for 5 min. The supernatant was removed, and the cells were resuspended in the culture medium. $5-10\times10^5$ cells were taken for cell purity analysis, wherein the result showed the proportion of KRT5 cells was ≥ 90%. Finally, all cells were plated in 30 T75 cell culture flasks for culture.

When the cells in the cell culture flasks grew to cover 85%-95% of the surface area of the culture flask, each culture flask was washed once with $1\times$DPBS, 5mL of recombinant trypsin was added, and the culture flasks were placed at 37°C for 5-15 min. After most of the cells became round and bright, adherent cells were gently detached by pipetting up and down with a 1 mL pipette and prepared into a single-cell suspension, and the digestion was terminated with an equal volume of DMEM medium. The cell suspension was collected, and then the bottom surface of the culture flask was rinsed with 5 mL of DMEM medium, and all the cell suspensions were collected. The collected cell suspensions were centrifuged at 1200 rpm for 5 min, the supernatant was removed, and the cells were washed for three times with normal saline for injection. Each washing step was performed as follows: 40 mL of $1\times$DPBS was used to resuspend the cell pellet, cells were centrifuged at 1200 rpm for 5 min, and the supernatant was removed. Then the cells were resuspended with 16 mL of normal saline for injection to prepare into a finished formulation, i.e., the REGEND001 autologous cell deliverable formulation.

Example 2 Preparation of REGEND001 autologous cell deliverable formulation

This example provides a process for the preparation of clinical-grade autologous bronchial basal cells, specifically including the following steps:

ex vivo bronchoscopic brushed-off biopsy tissues were prepared, and in this example, tissues from two different sites of the bronchus were selected. Selecting tissues from two or more sites is to ensure the success rate of separation.

A tissue digestion solution and a stop buffer were prepared. The tissue digestion solution comprised 99 v% of DMEM/F12, 1-20 ng/mL of DNase, 0.1-4 mg/mL of protease type XIV, and 10-200 ng/mL of trypsin. The stop buffer comprised 90 v% of DMEM and 10 v% of FBS.

The brushed-off biopsy tissues were digested with the tissue digestion solution. The digestion was terminated with the stop buffer, and digested cells were collected. The cells after enzyme digestion were subjected to bacterial detection and mycoplasma detection, and then part of the digested cells were cryopreserved.

A culture medium was prepared, wherein the culture medium comprised 225 mL of DMEM, 225 mL of F12, 20-70 mL of FBS, 0.2-2 mM of L-glutamine, 1-14 ng/mL of insulin, 0.1-1 ng/mL of epidermal growth factor, 5-30 μg/mL of adenine, and 2-20 μg/mL of hydrocortisone.

Irradiation-inactivated feeder cells in T25 cell culture flasks and cultured in a cell incubator (37°C, 5% $CO_2$) for 1-2 days, wherein the cells should have a density that the cells covered 50-70% of the bottom surface of the culture flask after 1-2 days.

Some of the digested cells were taken, and the cells derived from respective site were resuspended and plated into one T25 culture flask pre-coated with feeder cells, 200 ng/mL of gentamicin was added, and the cells were culture at 37°C with a $CO_2$ concentration of 4%-8%. The culture medium was replaced every other day. The cells were collected and cyropreserved when the cells grew to form clones wherein 80% or more of the clones contained 40-100 cells and clones of grade A and grade B were observed in three fields of view among randomly selected five fields of view.

The clones can be classified as grades A, B, and C depending on the morphology. The clones of grade A have a regular, round and smooth outline, clear boundary, and are closely arranged and have uniform sizes. The clones of grade B have a roughly regular outline, smooth and clear boundary, and most cells in clones are closely arranged and have uniform sizes, yet a small amount of the cells have a slightly larger size and are slightly loose arranged. The clones of grade C have irregular outlines and unclear boundary, and the cells in the clones are loosely arranged and have large sizes.

After the arrangement of the product preparation was confirmed, the cryopreserved cells were taken out (one tube for each site), and were quickly thawed in a 37°C water bath. All the liquids were collected in a 15mL tube, into which 2mL of recovery solution pre-warmed at 37°C was added dropwise, and the tube was centrifuged at 1200 rpm for 5 min. Supernatant was removed, and the cells were resuspended in the culture medium, and then cultured in T25 cell culture flasks pre-coated with feeder cells.

When the thawed cells grew to cover 50%-90% of the surface area of the cell culture flask, first passage was performed as follows: the cell culture supernatant was removed, and the cells were washed once with $1\times$ DPBS, and digested with 2 mL of recombinant trypsin at 37°C for 5-10 min. After most of the cells became round and bright, adherent cells were detached by pipetting up and down and prepared into a single-cell suspension. The digestion was terminated with an equal volume of DMEM medium, and the cell suspension was collected, centrifuged at 1200 rpm for 5 min. Supernatant was removed, and the cells were resuspended in the culture medium. Finally the resuspended cells were plated onto one T75 cell culture flask pre-coated with feeder cells at a density of $5\text{-}10\times10^3$ cells/cm$^2$. The culture medium was replaced every other day.

2nd passage was carried out when the first passaged cells grew to cover 50%-90% of the bottom surface area of the culture flask. The culture supernatant was removed, and the cells were washed once with $1\times$DPBS, and digested with 5mL of recombinant trypsin at 37°C for 5-10 min. Adherent cells were detached by pipetting up and down and prepared into a single-cell suspension after most of the cells became round and bright. The digestion was terminated with an equal volume of DMEM medium, and the resulting cell suspension was collected and centrifuged at 1200 rpm for 5 min. Supernatant was removed, and the cells are re-suspended in the culture medium. 2 mL of cell culture supernatant and $3\text{-}16\times10^5$ cells were taken to carry out tests, wherein bacterial detection and mycoplasma detection of gave negative results, HOPX positive percentage in biological petency test was $\geq$30%, cell identification test confirmed cells were KRT5 positive, and cell purity analysis showed KRT5 cell percentage was $\geq$ 90%. Meanwhile the re-suspended cells were plated onto three T75 cell culture flasks pre-coated with feeder cells at a density of $5\text{-}10\times10^3$ cells/cm$^2$, and the culture medium was replaced every other day.

When the second passaged cells grew to cover 50%-90% of the bottom surface area of the culture flask, 3rd passage was performed as follows: for cells in each flask, the culture supernatant was removed, and the cells were washed once with $1\times$DPBS, and digested with 5mL of recombinant trypsin at 37°C for 5-10 min. After most of the cells became round and bright, adherent cells were detached by pipetting up and down and prepared into a single-cell suspension. The digestion was terminated with an equal volume of DMEM medium. The cell suspension was collected and centrifuged at 1200 rpm for 5 min. Supernatant was removed, and the cells were resuspended in the culture medium. The re-suspended cells were plated onto ten T75 cell culture flasks pre-coated with feeder cells at a density of $5\text{-}10\times10^3$ cells/cm$^2$, and the culture medium was replaced every other day.

When the 3rd passaged cells grew to cover 50%-90% of the bottom surface area of the culture flask, the fourth passage was performed as follows: for cells in each flask, the culture supernatant was removed, and the cells were washed once with $1\times$DPBS, and digested with 5mL of recombinant trypsin at 37°C for 5-10 min. After most of the cells became round and bright, adherent cells were detached by pipetting up and down and prepared into a single-cell suspension. The digestion was terminated with an equal volume of DMEM medium. The cell suspension was collected, and centrifuged at 1200 rpm for 5 min. Supernatant was removed and the cells were resuspended in the culture medium. $1\text{-}10\times10^5$ cells were taken for biological potency test, wherein the HOPX positive percentage was $\geq$30%. Meanwhile, the resuspended cells were plated onto 30 T75 cell culture flasks pre-coated with feeder cells at a density of $5\text{-}10\times10^3$ cells/cm$^2$, and the culture medium was replaced every other day.

The feeder cells were removed when the fourth passaged cells grew to cover 50%-90% of the bottom surface area of the culture flask. The operation was performed as follows: the culture supernatant in each culture flask was removed, and each culture flask was washed once with $1\times$DPBS, 5mL of recombinant trypsin was added, and the culture flask was placed at 37°C for 1-2 min. The feeder layer cells were detached by pipetting up and down, washed again with $1\times$DPBS, and digested with 5mL of recombinant trypsin. After most of the cells became round and bright, adherent cells were detached by pipetting up and down and prepared into a single-cell suspension. The digestion was terminated with an equal volume of DMEM medium. The cell suspension was collected, and centrifuged at 1200 rpm for 5 min. Supernatant was removed, and the cells were resuspended in the culture medium. $5\text{-}10\times10^5$ cells were taken for cell purity analysis, and the result showed the proportion of KRT5 cells was >90%. Finally, all cells were plated in 30 T75 cell culture flasks for culture.

When the cells in the cell culture flasks grew to cover 85%-95% of the surface area of the culture flask, each culture flask was washed once with $1\times$DPBS, 5mL of recombinant trypsin was added, and the culture flasks were placed at 37°C for 5-15 min. After most of the cells became round and bright, adherent cells were gently detached by pipetting up and down with a 1 mL pipette and prepared into a single-cell suspension, and the digestion was terminated with an equal volume of DMEM medium. The cell suspension was collected, and then the bottom surface of the culture flask was rinsed with 5 mL of DMEM medium, and all the cell suspensions were collected. The cell suspensions were centrifuged at 1200 rpm for 5 min, the supernatant was removed, and the cells were washed for three times with noraml saline for injection. Each washing step was performed as follows: 40 mL of 1×DPBS was used to resuspend the cell pellet, cells were centrifuged at 1200 rpm for 5 min, and the supernatant was removed. Then the cells were resuspended with 16 mL of normal saline for injection to prepare into a finished formulation, i.e., the REGEND001 autologous cell deliverable formulation.

Example 3 Process of REGEND001 autologous cell deliverable formulation for the treatment of idiopathic pulmonary fibrosis

1) the process was carried out by the following specific steps:

(1) sites where the formulation is delivered back are 14 pulmonary subsegments (segmental bronchi) of the lingual lobe of the left lung, the inferior lobe of the left lung, the middle lobe of the right lung, and the inferior lobe of the right lung, specifically the lateral segmental bronchus of the middle lobe of the right lung, the medial segmental bronchus of the middle lobe of the right lung, the apical segmental bronchus of the inferior lobe of the right lung, the medial basal segmental bronchus of the right lung, the anterior basal segmental bronchus of the right lung, the lateral basal segmental bronchus of the right lung, the posterior basal segmental bronchus of the right lung, the superior segmental bronchus of the lingual lobe of the left lung, the inferior segmental bronchus of the lingual lobe of the left lung, the apical segmental bronchus of the inferior lobe of the left lung, the anterior basal segmental bronchus of the left lung, the medial basal segmental bronchus of the left lung, the lateral basal segmental bronchus of the left lung, and the posterior basal segmental bronchus of the left lung. No formulation is delivered to all of bronchial segments of the superior lobe of the left lung and the superior lobe of the right lung.

(2) 14 mL of the 16 mL REGEND001 autologous cell deliverable formulation prepared in Example 1 or 2 (it was tested that the 14 mL REGEND001 autologous cell deliverable formulation contained (4-300) $\times 10^6$ bronchial basal cells, preferably (2-30) $\times 10^7$ bronchial basal cells) was taken, and diluted to 42 mL with normal saline. The diluted REGEND001 autologous cell deliverable formulation was transferred to a syringe, which was connected to a catheter in a bronchoscopic operating channel, and injected into each pulmonary subsegment via a bronchofiberscope, with 3mL formulation injected into each pulmonary segment entrance (a pulmonary subsegment); specifically, 3 mL of the REGEND001 autologous cell deliverable formulation was injected into each pulmonary segment with the syringe, followed by injecting 5 mL of air, which was used to push the REGEND001 autologous cell deliverable formulation into the distal pulmonary subsegment.

(3) It would take about 1.5 hours to establish tight adhesion between the cells and the inflammatory and wounded areas of the lungs, and after completion of transplantation, the subjects were required to lie in horizontal position for about two hours.

(4) Fasting for food and water was required within 2 hours after treatment, the subjects shall avoid coughing as much as possible, and codeine can be given orally if necessary.

2) Treatment course

The REGEND001 autologous cell deliverable formulation was proposed to be used once, the treatment was divided into two parts, wherein the first part was the collection, isolation and culture of bronchial basal cells (i.e. Example 1 or 2), the second part was once transplantation and delivery of the REGEND001 autologous cell deliverable formulation (the delivery was done only once during the whole course of treatment). The treatment course would take totally 4-8 weeks from the collection and isolation of bronchial basal cells to the formulation administration, followed by a 24-week follow-up observation period in which subjects needed to go to the hospital to receive corresponding tests, including safety and efficacy tests.

Safety tests included blood routine examination, urine routine examination, blood biochemistry, electrocardiogram and lung tumor marker detection.

Efficacy tests included pulmonary function tests (including the actual measured value of diffusing capacity of carbon monoxide (DLCO), the actual measured value/the predicted value of diffusing capacity of carbon monoxide (DLCO%), forced vital capacity FVC), six-minute walk test, and St. George's Respiratory Questionnaire (SGRQ).

3) The following is pulmonary function data of four patients with IPF before and after treatment.

According to the guidelines in the diagnosis of idiopathic pulmonary fibrosis (IPF) issued jointly by the four international respiratory societies, the American Thoracic Society (ATS), the European Respiratory Society (ERS), the Japanese Respiratory Society (JRS) and the Latin American Thoracic Association (ALAT), in the American Journal of Respiratory Critical Care in September, 2018 (references: 1. Raghu G, et al. Diagnosis of Idiopathic Pulmonary Fibrosis. An Official ATS/ERS/JRS/ALAT Clinical Practice Guideline. Am J Respir Crit Care Med Published Online: Sep 2018; 2. Raghu G, et al. ATS/ERS/JRS/ALAT Committee on Idiopathic Pulmonary Fibrosis. An Official ATS/ERS/JRS/ALAT Statement: Idiopathic Pulmonary Fibrosis: Evidence-based Guidelines for Diagnosis and Management. Am J Respir Crit Care Med Published Online: Mar 2011): IPF is a specific form of chronic progressive fibrotic interstitial pneumonia with unknown causes, which is confined to the lungs. The diagnostic criteria are: 1. exclusion of other interstitial lung diseases (ILD) (e.g., ILD associated with home or occupational environmental exposure, ILD associated with connective tissue disease, ILD associated with drug toxicity); 2. HRCT is manifested as UIP; 3. Patients with lung tissue specimens can be diagnosed by combining HRCT and histological features. (Where one of the above items 2 and 3 can be satisfied). Patients with idiopathic pulmonary fibrosis have an impaired pulmonary ventilation function (pulmonary diffusion function) and the actual measured value/the predicted value of DLCO (DLCO%) is less than 80%. (Clinical pulmonary diffusion function classification: normal: 80% ≤ DLCO/predicted (DLCO%); mild: 60% ≤ DLCO/predicted (DLCO%) < 80%; moderate: 40% ≤ DLCO/predicted (DLCO%) < 60%; severe: DLCO/predicted (DLCO%) < 40%).

Case 1:

Male, 66 years old, height 172 cm, weight 83 kg, suffering from recurrent cough and sputum with shortness of breath for 2 years, clinically diagnosed as idiopathic pulmonary fibrosis, with moderate diffusion dysfunction. Meanwhile suffered from hypertension for 7 years and type 2 diabetes for 7 years. The patient was treated with the REGEND001 autologous cell deliverable formulation prepared in Example 1 at a dose of $1.13 \times 10^6$ bronchial basal cells/kg.

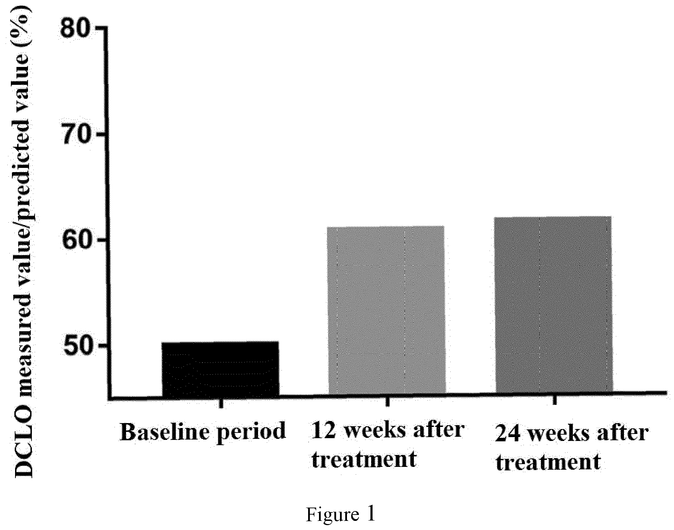

Pulmonary ventilation function (DLCO) before/predicted (DLCO%) (i.e., actual measured value/predicted value %) was 50.0% at a baseline, and was 60.7% at 12 weeks after transplantation treatment, indicating an improvement of 21.4% compared to baseline; and was 61.5% at 24 weeks after treatment, indicating an improvement of 23.0% compared to baseline. The diffusion function is improved significantly after treatment (see Table 1 and Figure 1).

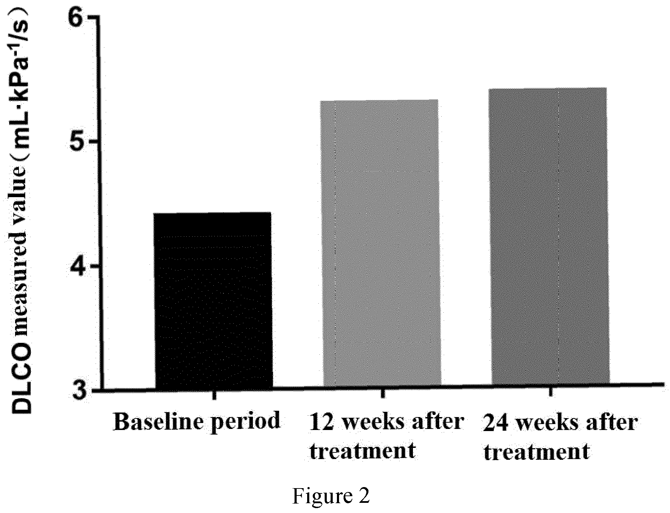

Pulmonary ventilation function, actual measured value of DLCO (mL·kPa$^{-1}$/s) was 4.40 at the baseline, 5.29 at 12 weeks after treatment, and 5.37 at 24 weeks after treatment. The diffusion function is improved significantly after treatment (see Figure 2).

The result of 6-minute walk test was 330 m at the baseline, and 523 m at 12 weeks after treatment (193 m or 58.48% increase than the baseline). The motor function status is improved significantly.

St. George's Respiratory Questionnaire (SGRQ) was 17.39 at the baseline, 9.57 at 12 weeks after treatment (7.82 or 44.97% decrease than the baseline). The treatment on quality of life is improved significantly.

Case 2:

Male, 64 years old, height 172 cm, weight 86 kg, suffering from recurrent cough and sputum, clinically diagnosed as idiopathic pulmonary fibrosis with severe diffusion dysfunction for 4 years of disease duration. Meanwhile suffered from coronary heart disease for 4 years and hypertension for 4 years. The patient was treated with the REGEND001 autologous cell deliverable formulation prepared in Example 2 at a dose of $0.71 \times 10^6$ bronchial basal cells/kg.

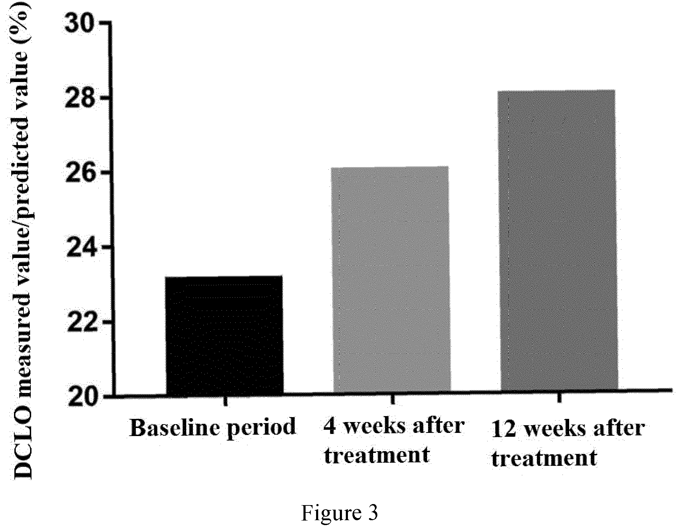

Pulmonary ventilation function DLCO before/predicted (DLCO%) (i.e., actual measured value/predicted value %) was 23.1% at a baseline, and was 26% at 4 weeks after treatment, and 28% at 12 weeks after treatment, indicating a significant improvement in diffusion function compared to baseline (see Table 1 and Figure 3).

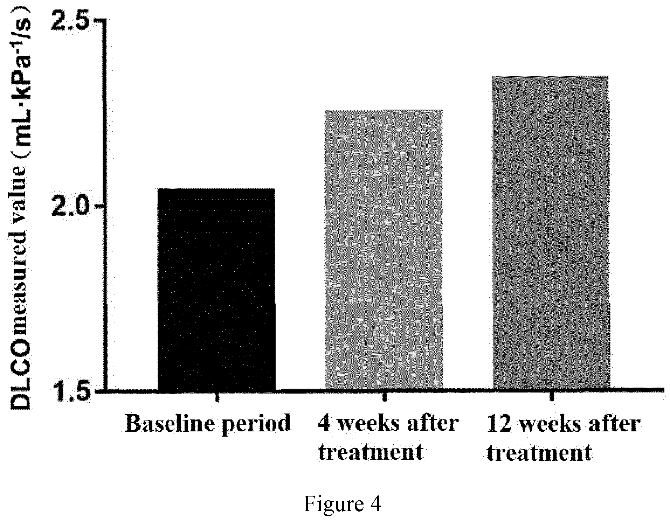

The pulmonary ventilation function, actual measured value of DLCO (mL·kPa$^{-1}$/s) was 2.04 at the baseline, 2.25 at 4 weeks after treatment, and 2.34 at 12 weeks after treatment, indicating significant improvement in diffusion function than the baseline (see Figure 4).

There was no abnormal or abnormally increased change in urine routine examination and blood biochemistry before and after treatment.

Case 3:

Female, 60 years old, height 156 cm, weight 55 kg, suffering from recurrent cough and sputum for more than 8 years, clinically diagnosed as idiopathic pulmonary fibrosis, which was severe with undetectable diffusion function. Meanwhile suffered from Hashimoto's thyroiditis. The patient was treated with the REGEND001 autologous cell deliverable formulation prepared in Example 2 at a dose of $3.84 \times 10^6$ bronchial basal cells/kg.

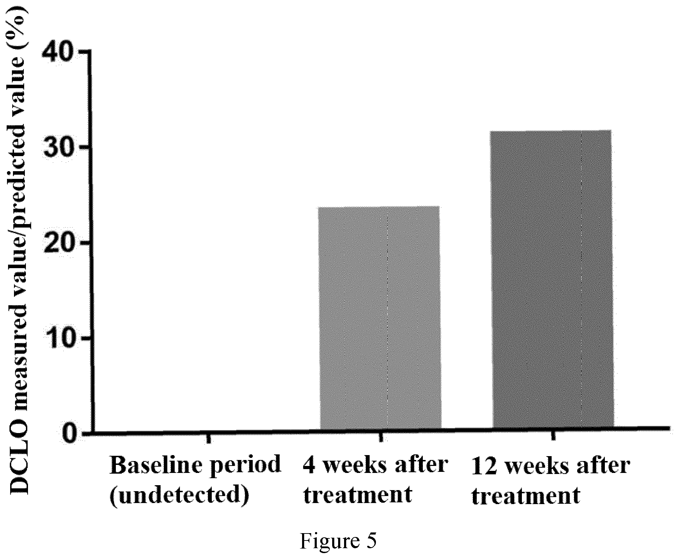

DLCO before/predicted (DLCO%) (i.e. actual measured value/predicted value %) was not detected at baseline, and this value was 23.2% at 4 weeks after treatment, and 31% at 12 weeks after treatment, indicating significant improvement in diffusion function (see Table 1 and Figure 5).

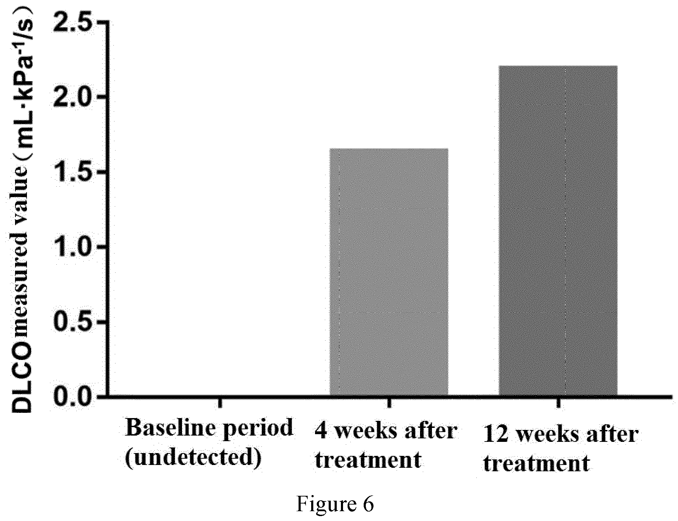

The DLCO actual measured value (mL·kPa$^{-1}$/s) was not measured at baseline, was 1.64 at 4 weeks after treatment and 2.19 at 12 weeks after treatment, indicating a significant improvement in diffusion function (see Figure 6).

It is apparent that the foregoing examples are merely examples for clear description and are not intended to limit the embodiments. For a person of ordinary skill in the art, variations or changes in other different forms can be made based on the above description. It is not necessary or possible to be exhaustive of all embodiments here. The obvious variations or changes derived therefrom still fall within the protection scope of the present application.

**Claims**

1. Use of a REGEND001 autologous cell deliverable formulation in the preparation of a product, wherein the product has a function of improving the pulmonary function in patients with idiopathic pulmonary fibrosis.

2. Use of a REGEND001 autologous cell deliverable formulation in the preparation of a product, wherein the product has a function of improving the pulmonary ventilation function in patients with idiopathic pulmonary fibrosis.

3. Use of a REGEND001 autologous cell deliverable formulation in the preparation of a product for improving and/or treating the pulmonary ventilation function in humans.

4. Use of a REGEND001 autologous cell deliverable formulation in the preparation of a product for improving the pulmonary diffusion function in humans.

5. Use of a REGEND001 autologous cell deliverable formulation in the preparation of a product for improving the motor function in humans.

6. The use according to any one of claims 1-5, wherein the REGEND001 autologous cell deliverable formulation is administrated to any pulmonary subsegment of the lingual lobe of the left lung, the inferior lobe of the left lung, the middle lobe of the right lung, and the inferior lobe of the right lung.

7. The use according to any one of claims 1-5, wherein the REGEND001 autologous cell deliverable formulation is administered at a dose of $0.1\text{-}10\times10^6$ cells/kg, preferably $1\text{-}10\times10^6$ cells/kg.

8. Use of a REGEND001 autologous cell deliverable formulation in the treatment of idiopathic pulmonary fibrosis.

9. A REGEND001 autologous cell deliverable formulation, wherein the REGEND001 autologous cell deliverable formulation is a finished cell formulation that is prepared by collecting, isolating *in vitro,* culturing and amplification of autologous bronchial basal cells of patients.

10. The REGEND001 autologous cell deliverable formulation according to claim 9, wherein the finished formulation contains bronchial basal cells suspended in normal saline for injection, and each 14 mL of the REGEND001 autologous cell deliverable formulation contains $(4\text{-}300) \times 10^6$ bronchial basal cells, preferably $(2\text{-}30) \times 10^7$ bronchial basal cells.

11. The REGEND001 autologous cell deliverable formulation according to claim 9 or 10, wherein the REGEND001 autologous cell deliverable formulation is prepared by the following steps:

    tissue preparation: getting an ex vivo bronchoscopic brushed-off biopsy tissue from at least one site;
    enzyme digestion: digesting the tissue with a tissue digestion solution, and collecting cells after terminating the digestion with a stop buffer;
    plating and amplification culture: plating a part of the digested cells in a culture flask pre-coated with feeder cells to perform primary culture, collecting the primary cultured cells to perform amplification culture in a culture flask pre-coated with feeder cells, followed by passage culture when the cells grow to cover 50%-90% of the surface area of the culture flask, or when the cells grow to form clones wherein 80% or more of the clones contain 40-100 cells and clones of grade A and grade B are observed in three fields of view among randomly selected five fields of view; wherein the clones of grade A have a regular, round and smooth outline, and clear boundary, and cells are closely arranged in clones and have uniform sizes; and the clones of grade B have a roughly regular outline, smooth and clear boundary, and most cells in the clones are closely arranged and have uniform sizes, yet a small amount of the cells have a slightly larger size and are slightly loose arranged;
    cell collecting: when passaged cells grow to cover 85%-95% of the surface area of the culture flask, digesting and collecting adherent cells, and washing.

12. The REGEND001 autologous cell deliverable formulation according to claim 11, wherein the tissue digestion solution comprises 99 v% of DMEM/F12, 1-20 ng/mL of DNase, 0.1-4 mg/mL of protease type XIV, and 10-200 ng/mL of trypsin; and the digestion is performed at a temperature of 37°C for a duration of 0.5-2 h.

13. The REGEND001 autologous cell deliverable formulation according to claim 11, wherein said primary culture and amplification culture are carried out with a culture medium comprising 225 mL of DMEM, 225 mL of F12, 20-70 mL of fetal bovine serum (FBS), 0.2-2 mM of L-glutamine, 1-14 ng/mL of insulin, 0.1-1 ng/mL of epidermal growth factor, 5-30 μg/mL of adenine, and 2-20 μg/mL of hydrocortisone.

14. The REGEND001 autologous cell deliverable formulation according to claim 11, wherein the stop buffer comprises 90 v% of DMEM and 10 v% of FBS.

15. The REGEND001 autologous cell deliverable formulation according to claim 11, wherein the cells yielded in the steps of enzyme digestion and primary culture are cryopreserved; preferably before cryopreservation, bacterial detection and mycoplasma detection are carried out; and preferably the cryopreserved cells are rapidly thawed and used to perform the primary culture and/or amplification culture.

16. A method for treating idiopathic pulmonary fibrosis, comprising administering a REGEND001 autologous cell deliverable formulation to a subject with idiopathic pulmonary fibrosis, wherein the REGEND001 autologous cell deliverable formulation is a finished cell formulation prepared by collecting, isolating *in vitro,* culturing and amplification of autologous bronchial basal cells of patients.

17. The method for treating idiopathic pulmonary fibrosis according to claim 16, wherein the administering comprises delivering the REGEND001 autologous cell deliverable formulation to one or more of 14 pulmonary subsegments selected from the lingual lobe of the left lung, the inferior lobe of the left lung, the middle lobe of the right lung, and the inferior lobe of the right lung, and the 14 pulmonary subsegments is respectively the lateral segmental bronchus of the middle lobe of the right lung, the medial segmental bronchus of the middle lobe of the right lung, the apical segmental bronchus of the inferior lobe of the right lung, the medial basal segmental bronchus of the right lung, the anterior basal segmental bronchus of the right lung, the lateral basal segmental bronchus of the right lung, the posterior basal segmental bronchus of the right lung, the superior segmental bronchus of the lingual lobe of the left lung, the inferior segmental bronchus of the lingual lobe of the left lung, the apical segmental bronchus of the inferior lobe of the left lung, the anterior basal segmental bronchus of the left lung, the medial basal segmental bronchus of the left lung, the lateral basal segmental bronchus of the left lung, and the posterior basal segmental bronchus of the left lung.

18. The method for treating idiopathic pulmonary fibrosis according to claim 17, wherein the REGEND001 autologous cell deliverable formulation comprises bronchial basal cells suspended in normal saline for injection, and each 14 mL of the REGEND001 autologous cell deliverable formulation comprises $(4\text{-}300)\times 10^6$ bronchial basal cells, preferably $(2\text{-}30)\times 10^7$ bronchial basal cells.

19. The method for treating idiopathic pulmonary fibrosis according to claim 18, comprising diluting 14 mL of the REGEND001 autologous cell deliverable formulation to 42 mL with normal saline, and then injecting the diluted solution into one or more of the 14 pulmonary subsegments by using a syringe.

20. The method for treating idiopathic pulmonary fibrosis according to claim 16, wherein the subject has moderate or severe diffusion dysfunction.

21. The method for treating idiopathic pulmonary fibrosis according to claim 16, wherein the subject further suffers from one or more diseases or conditions selected from: hypertension, type 2 diabetes, coronary artery disease, Hashimoto's thyroiditis, and pulmonary emphysema.

22. The method for treating idiopathic pulmonary fibrosis according to claim 16, wherein the REGEND001 autologous cell deliverable formulation is administered at a dose of $0.1\text{-}10\times 10^6$ bronchial basal cells/kg, preferably $1\text{-}10\times 10^6$ bronchial basal cells/kg.

Figure 1

Figure 2

Figure 3

Figure 4

Figure 5

Figure 6

| INTERNATIONAL SEARCH REPORT | | International application No. |
|---|---|---|
| | | **PCT/CN2021/106176** |

**A. CLASSIFICATION OF SUBJECT MATTER**

C12N 5/071(2010.01)i; A61L 27/38(2006.01)i; A61K 35/42(2015.01)i; A61P 11/00(2006.01)i

According to International Patent Classification (IPC) or to both national classification and IPC

**B. FIELDS SEARCHED**

Minimum documentation searched (classification system followed by classification symbols)

C12N,A61L,A61K,A61P

Documentation searched other than minimum documentation to the extent that such documents are included in the fields searched

Electronic data base consulted during the international search (name of data base and, where practicable, search terms used)

DWPI, SIPOABS, CNABS, USTXT, JPTXT, EPTXT, WOTXT, CNTXT, CNKI, ISI WEB OF SCIENCE, PUBMED: 支气管基底层细胞, 肺, 支气管, 基底层细胞, 基底细胞, 纤维化, 肺, basal layer cells, basilar membrane cells, bronchial basal cells, bronchial epithelial basal cells, fibrosis, lungs, SOX9, P63, Krt5

**C. DOCUMENTS CONSIDERED TO BE RELEVANT**

| Category* | Citation of document, with indication, where appropriate, of the relevant passages | Relevant to claim No. |
|---|---|---|
| PX | CN 111944737 A (REGEND THERAPEUTICS CO., LTD.) 17 November 2020 (2020-11-17)<br>see claims 1-19 | 1-7, 9-15 |
| Y | CN 111297907 A (LIU, Siwei et al.) 19 June 2020 (2020-06-19)<br>see claims 1-8, embodiment | 10-15 |
| Y | CN 105916978 A (YALE UNIVERSITY) 31 August 2016 (2016-08-31)<br>see claims 1-32 | 1-4, 6, 7 |
| A | SU 1820275 A1 (KIEV FTIZIATRY PULMONOLOGY RES INST) 07 June 1993 (1993-06-07)<br>see abstract | 1-7, 9-15 |
| X | 程林 等 (CHENG, Lin et al.). "自体支气管基底层细胞治疗慢性阻塞性肺疾病的小样本探索性研究 (A Small Sample Exploratory Study of Autologous Bronchial Basal Cells for the Treatment of Chronic Obstructive Pulmonary Disease)"<br>《重庆医学》 (Chongqing Medicine), Vol. 48, No. 23, 31 December 2019 (2019-12-31), ISSN: 1671-8348,<br>pages 4012-4016, see abstract, 1.2 and 3 in part | 5,9 |

☑ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **27 September 2021** | **13 October 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2021/106176**

### C.    DOCUMENTS CONSIDERED TO BE RELEVANT

| Category* | Citation of document, with indication, where appropriate, of the relevant passages | Relevant to claim No. |
|---|---|---|
| Y | 程林 等 (CHENG, Lin et al.). "自体支气管基底层细胞治疗慢性阻塞性肺疾病的小样本探索性研究 (A Small Sample Exploratory Study of Autologous Bronchial Basal Cells for the Treatment of Chronic Obstructive Pulmonary Disease)" 《重庆医学》 (Chongqing Medicine), Vol. 48, No. 23, 31 December 2019 (2019-12-31), ISSN: 1671-8348, pages 4012-4016, see abstract, 1.2 and 3 in part | 1-4, 6, 7, 10-15 |
| A | Yang et al. "Stem/progenitor cells in endogenous repairing responses: new toolbox for the treatment of acute lung injury" Journal of translational medicine, Vol. 14, 11 February 2016 (2016-02-11), ISSN: 1479-5876, pp. 1-14, see entire document | 1-7, 9-15 |
| A | Jennifer R. Peters-Hall et al. "Long-term culture and cloning of primary human bronchial basal cells that maintain multipotent differentiation capacity and CFTR channel function" AMERICAN JOURNAL OF PHYSIOLOGY-LUNG CELLULAR AND MOLECULAR PHYSIOLOGY, Vol. 315, No. 2, 03 May 2018 (2018-05-03), ISSN: 1522-1504, pages L313–L327, see the whole document | 1-7, 9-15 |

Form PCT/ISA/210 (second sheet) (January 2015)

**INTERNATIONAL SEARCH REPORT**

| International application No. |
| --- |
| **PCT/CN2021/106176** |

**Box No. II**      **Observations where certain claims were found unsearchable (Continuation of item 2 of first sheet)**

This international search report has not been established in respect of certain claims under Article 17(2)(a) for the following reasons:

1. ☑   Claims Nos.: **8**, **16-22**
   because they relate to subject matter not required to be searched by this Authority, namely:

   [1]   A treatment method for the human body or animal body

2. ☐   Claims Nos.:
   because they relate to parts of the international application that do not comply with the prescribed requirements to such an extent that no meaningful international search can be carried out, specifically:

3. ☐   Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2021/106176**

| Patent document cited in search report | | | Publication date (day/month/year) | Patent family member(s) | | | Publication date (day/month/year) |
|---|---|---|---|---|---|---|---|
| CN | 111944737 | A | 17 November 2020 | WO | 2020228819 | A1 | 19 November 2020 |
| CN | 111297907 | A | 19 June 2020 | None | | | |
| CN | 105916978 | A | 31 August 2016 | AU | 2015206684 | A1 | 23 June 2016 |
| | | | | EP | 3094720 | A1 | 23 November 2016 |
| | | | | JP | 2017506883 | A | 16 March 2017 |
| | | | | AU | 2015206684 | B2 | 25 March 2021 |
| | | | | CN | 112011500 | A | 01 December 2020 |
| | | | | KR | 20160104005 | A | 02 September 2016 |
| | | | | JP | 6702876 | B2 | 03 June 2020 |
| | | | | WO | 2015108893 | A1 | 23 July 2015 |
| | | | | US | 2016312190 | A1 | 27 October 2016 |
| | | | | CA | 2935590 | A1 | 23 July 2015 |
| | | | | JP | 2020072688 | A | 14 May 2020 |
| | | | | EP | 3094720 | A4 | 02 August 2017 |
| | | | | IL | 246352 | D0 | 31 August 2016 |
| SU | 1820275 | A1 | 07 June 1993 | None | | | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201910407062
- CN 111944737 A